Europäisches Patentamt

European Patent Office

Office européen des brevets

(19)

(11) Numéro de publication: **0 271 383 B1**

(12) ## FASCICULE DE BREVET EUROPEEN

(45) Date de publication de fascicule du brevet:
03.07.91

(51) Int. Cl.5: **G01N 1/14**

(21) Numéro de dépôt: **87402555.4**

(22) Date de dépôt: **12.11.87**

(54) **Dispositif de nettoyage d'une aiguille de prélèvement d'un liquide.**

(30) Priorité: **14.11.86 FR 8615833**

(43) Date de publication de la demande:
**15.06.88 Bulletin 88/24**

(45) Mention de la délivrance du brevet:
**03.07.91 Bulletin 91/27**

(84) Etats contractants désignés:
**AT BE CH DE ES FR GB IT LI LU NL SE**

(56) Documents cités:
**FR-A- 2 354 555**
**US-A- 3 719 086**
**US-A- 3 841 160**
**US-A- 3 911 749**
**US-A- 4 318 885**

**PATENT ABSTRACTS OF JAPAN, vol. 9, no. 182 (P-376)[1905], 27 juillet 1985; & JP-A-60 53 849 (HITACHI SEISAKUSHO K.K.) 27-03-1985**

(73) Titulaire: **ABX**
**16 rue Baudin**
**F-92300 Levallois-Perret(FR)**

(72) Inventeur: **Champseix, Henri**
**4 rue Audran**
**F-78360 Montesson(FR)**
Inventeur: **Champseix, Serge**
**29 bis, rue des Puiseux**
**F-78130 Les Mureaux(FR)**

(74) Mandataire: **Lepeudry-Gautherat, Thérèse et al**
**ARMENGAUD JEUNE CABINET LEPEUDRY 6, rue du Fg. St-Honoré**
**F-75008 Paris(FR)**

## Description

L'invention qui se rapporte au prélèvement d'un liquide dans un flacon, notamment au prélèvement du sang, concerne plus précisément un dispositif de nettoyage de l'aiguille servant à prélever ce liquide.

Dans les laboratoires d'analyses de biologie médicale, on sait que des appareils automatiques permettent la détermination d'un certain nombre de paramètres tels que le nombre de leucocytes et d'hématies, le dosage de l'hémoglobine etc..., à partir d'un échantillon de sang entier. Les échantillons de sang doivent donc subir des transferts, dont au moins un de ceux-ci consiste à en prélever dans un tube une petite quantité à l'aide d'une aiguille qui plonge dans le sang et en aspire un petit volume. A l'issue de cette opération et avant d'effectuer un nouveau prélèvement, il convient d'assurer un rinçage soigneux de l'aiguille, aussi bien intérieurement qu'extérieurement.

Jusqu'à présent il était habituel de tremper l'aiguille dans un liquide de rinçage, celui-ci pénétrant également à l'intérieur, puis de l'égoutter. Pour que le nettoyage soit efficace, il convenait ensuite d'essuyer l'aiguille, ce qui compliquait l'opération et introduisait une nouvelle cause de pollution. Pour éviter cet inconvénient et faire en sorte que le nettoyage intérieur de l'aiguille soit assuré correctement, on a pensé utiliser un bloc de rinçage fixe. Celui-ci est une sorte de réservoir, dans lequel vient tremper l'aiguille mobile par ailleurs reliée par un petit tube flexible à un système de pompage qui permet d'aspirer et de refouler dans l'intérieur de l'aiguille le liquide de rinçage et éventuellement un fluide de séchage. Mais il faut alors que la profondeur du réservoir corresponde à la longueur de l'aiguille à nettoyer, et que ladite aiguille soit munie d'un tube de raccordement suffisamment long pour pouvoir se déplacer dans ce réservoir, ce qui représente un encombrement important, et une mécanisation compliquée.

Dans le FR-A-2 354 555 est décrit un autre dispositif de nettoyage adapté à une pipette d'aspiration d'échantillon qui est également mobile entre une position basse où elle plonge dans un récipient d'échantillon pour le prélever et une position haute où son extrémité s'efface à l'intérieur d'une chambre de lavage, une solution de lavage étant injectée dans cette chambre, puis aspirée dans la pipette sans s'écouler par l'orifice ouvert inférieur de la chambre. Selon cette disposition le fluide de rinçage est aspiré à l'intérieur de la pipette dans le même sens que le prélèvement de l'échantillon. Cela présente l'inconvénient qu'il faut récupérer ledit fluide de rinçage du conduit d'aspiration, pour l'orienter vers un réservoir annexe de déchet, ce qui impose de créer un circuit en dérivation sur ce conduit avec la vanne d'aiguillage et la pompe d'aspiration appropriées. Un autre inconvénient de ce dispositif réside dans le fait que l'air aspiré dans la pipette avec le liquide de rinçage forme à l'intérieur de la pipette une émulsion qui rince moins correctement sa paroi interne, que la paroi externe qui est au contraire nettoyée uniquement par du liquide, alors qu'un bon nettoyage de l'intérieur de la pipette s'avèrerait plus important à obtenir que l'extérieur.

Dans ce cas également le fait de devoir déplacer la pipette ou l'aiguille de prélèvement nécessite une mécanisation particulière au détriment de l'encombrement.

Il existe cependant des systèmes dans lesquels l'aiguille reste en position fixe. Le nettoyage de l'extrémité de l'aiguille est alors assuré par des godets mobiles de rinçage qui viennent par au-dessous pour que l'aiguille trempe dans le liquide qu'ils contiennent, avant de s'escamoter à l'issue de l'opération de rinçage. Ce système impose une grande précision dans le déplacement des godets par rapport à l'aiguille, ce qui rend l'opération délicate. Tous ces dispositfs présentent aussi en commun l'inconvénient de nécessiter l'emploi d'une quantité non négligeable de liquide de nettoyage, au détriment de la rapidité d'exécution et du prix de revient.

Enfin, le document US-A- 4318 885 divulgue un dispositif de nettoyage d'une aiguille ayant toutes les caractéristiques présentes dans le préambule de la revendication 1 de la demande.

L'invention propose donc une nouvelle solution à ces problèmes, grâce à laquelle les inconvénients inhérents aux systèmes connus sont éliminés, solution qui présente en outre l'avantage d'être très aisément applicable à des appareils de prélèvement automatique à aiguille fixe.

Selon l'invention le dispositif de nettoyage d'une aiguille de prélèvement d'un liquide, notamment du sang, montée sur le bâti d'un appareil de prélèvement qui utilise un mécanisme mobile apte à venir en contact avec ladite aiguille, lequel mécanisme mobile comportant une chambre cylindrique de nettoyage ouverte vers l'extérieur destinée à être traversée par l'aiguille, ce dispositif donc est constitué d'un boîtier de rinçage coulissant sur l'aiguille fixe de prélèvement entre une position haute dans laquelle il dégage la partie inférieure de l'aiguille et la laisse tout à fait accessible pour un prélèvement et une position basse qu'il occupe lors du nettoyage où il coiffe toute la partie inférieure de l'aiguille, l'extrémité de celle-ci restant en retrait à l'intérieur de la chambre cylindrique et en ce que à la partie inférieure de ladite chambre est appliquée une dépression par un orifice qui aspire un fluide de rinçage injecté par le haut à l'intérieur de l'aiguille, lequel fluide assure un rinçage intérieur à contre-courant du sens de prélèvement du liquide, un second orifice, sur la partie supérieure de la chambre cylindrique permet d'injecter un fluide de rinçage qui assure le rinçage extérieur de l'aiguille.

Selon une caractéristique particulière de l'invention la partie supérieure du boîtier de rinçage est percée d'une chambre cylindrique traversée par l'aiguille et fermée par une pièce de guidage, au moins un joint faisant étanchéité avec l'aiguille étant disposé au fond de la chambre.

Selon encore une autre caractéristique de l'invention, le boîtier de rinçage est fixé à un coulisseau qui se déplace sur une colonne de guidage, fixe et parallèle à l'aiguille, la tige mobile d'un vérin fixe étant solidaire du coulisseau et une même potence fixée au bâti de l'appareil de prélèvement servant de support à la colonne et au vérin.

D'autres caractéristiques particulières et avantages de l'invention apparaîtront à la lecture de la description qui va suivre d'une forme de réalisation prise à titre d'exemple non limitatif et faisant référence aux dessins annexés qui représentent :

Figure 1 une vue schématique partielle en élévation du dispositif de nettoyage.

Figure 2 une vue en coupe à plus grande échelle du boîtier de rinçage.

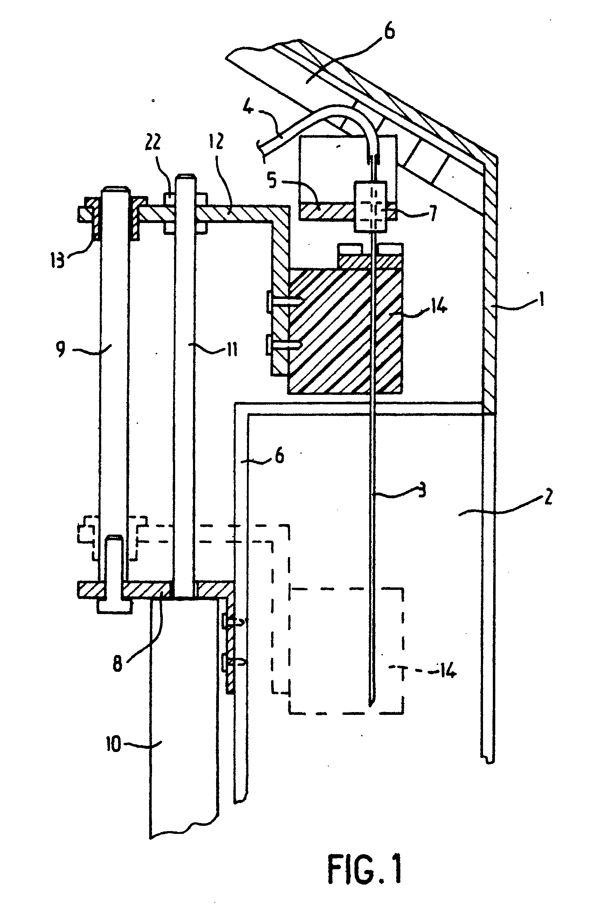

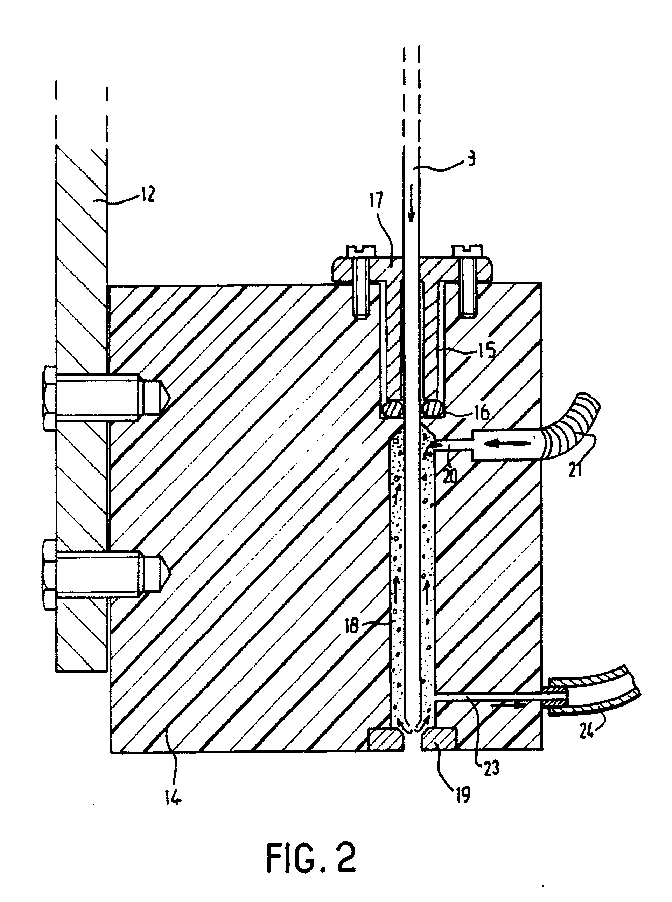

On a représenté à la figure 1 la partie avant 1 du bâti 6 d'un appareil de prélèvement et d'analyse, ménageant une alvéole 2 à l'intérieur de laquelle on maintient un flacon en position pour qu'une aiguille de prélèvement 3 pénètre à l'intérieur. L'extrémité supérieure de l'aiguille 3 reliée à l'appareil par un petit conduit flexible 4 est fixée à une équerre 5 solidaire du bâti 6, par l'intermédiaire d'un étrier flottant 7. Ce dernier soutient l'aiguille en position sensiblement verticale comme représentée, tout en lui laissant un faible jeu par rapport à son support. Sur le bâti 6 est fixé, à proximité de l'alvéole 2, une potence 8 qui sert de support à une colonne de guidage 9 parallèle à l'aiguille 3. A cette potence est également fixé sensiblement verticalement un vérin 10 dont la tige mobile 11 se déplace aussi parallèlement à l'aiguille. Sur la colonne de guidage 9 est engagé un coulisseau 12 qui se déplace sans frottement sur elle grâce à une bague de guidage 13. Le coulisseau est fixé par un organe de clipsage 22 à l'extrémité de la tige 11 du vérin 10, qui assure son déplacement d'un bout à l'autre de la colonne. Ce coulisseau dont l'extrémité déborde à l'intérieur de l'alvéole de prélèvement sert de support à un boîtier de rinçage ou de nettoyage 14 qui est donc mobile avec lui et apte à se déplacer avec lui de bas en haut et de haut en bas en coulissant sur l'aiguille de prélèvement 3. Le boîtier de rinçage représenté plus précisément à la figure 2 est traversé verticalement par une ouverture permettant le passage de l'aiguille 3. A la partie supérieure est percée une chambre cylindrique 15 traversée par l'aiguille au fond de laquelle sont logés deux joints toriques 16 faisant étanchéité. Une pièce de guidage 17 est en outre rapportée en haut de la chambre 15 pour guider l'aiguille et comprimer le joint. A la partie inférieure est prévue une chambre cylindrique de nettoyage 18 dont la partie inférieure est ouverte vers l'extérieur, l'ouverture étant bordée d'un embout profilé 19. La base de cette chambre communique par un orifice 23 avec un conduit d'aspiration 24 lui-même relié à une pompe non représentée.

Le sommet de la chambre 18 communique également par un orifice additionnel 20 avec un conduit d'injection 21 également relié à une pompe non représentée, ce conduit n'assurant que l'introduction de fluide au sommet de la chambre 18, dans le sens de la flèche, à l'inverse du conduit 24 qui n'exerce qu'une aspiration de fluide à la partie basse de ladite chambre.

La figure 1 montre le boîtier de rinçage 14 maintenu en position haute par la tige 11 déployée du vérin. La partie d'aiguille 3 située dans l'alvéole 2 est donc tout à fait accessible à l'utilisateur qui peut lui présenter un flacon en vue d'un prélèvement. Quand le sang a été prélevé par le conduit 4, on agit sur le vérin 10 dans le sens de la rétraction de la tige 11 et de la descente du coulisseau 12. Le boîtier de rinçage 14 effectue alors son mouvement de descente pendant lequel d'une part, du fluide de rinçage est injecté par le conduit 21 à la partie supérieure de la chambre 18 au niveau de l'orifice 20 et, d'autre part, simultanément une aspiration est assurée dans le conduit 24 qui crée une dépression à la base de la chambre 18 par l'orifice 23. Par conséquent, pendant la descente du boîtier de rinçage est assuré en quelque sorte un premier nettoyage de l'extérieur de l'aiguille sur toute sa hauteur qui évite une saturation de sang dans le dispositif et les circuits de rinçage.

Puis le boîtier de rinçage 14 atteint la position basse représentée à la figure 2 et en pointillé à la figure 1, position selon laquelle il coiffe toute la partie inférieure de l'aiguille 3. L'extrémité inférieure de l'aiguille 3 se trouve alors légèrement en retrait de l'orifice inférieur du boîtier, à l'intérieur de la chambre 18. Dès que cette position basse est atteinte l'injection de fluide par le conduit 21 est interrompue, mais l'aspiration est maintenue par le conduit 24 alors que simultanément on assure l'injection de fluide de rinçage, mais par le haut dans le conduit 4 et par conséquent à l'intérieur de l'aiguille 3 et à contre-courant du sens de prélèvement du sang. Du fait de cette injection par le haut le fluide va rincer l'intérieur de l'aiguille 3 sur toute sa hauteur, puis déboucher à la partie basse à proximité de l'embout 19. Il sera alors soumis à l'aspiration exercée par l'orifice 23, de ce fait le fluide ne s'écoulera pas en dehors du boîtier 14. La position et la forme de l'embout profilé 19 sont adaptées pour coopérer avec l'extrémité inférieure de l'aiguille 3 en vue d'éviter l'écoulement de fluide de rinçage hors du boîtier. A l'issue de cette opération le boîtier 14 remonte en position haute.

Au cours de cette opération de remontée, l'injection de fluide est interrompue par le conduit 4 mais par contre, elle est rétablie par le conduit 21, et puisque l'aspiration par le conduit 24 est maintenue, un nettoyage complémentaire de sécurité de la partie extérieure de l'aiguille est assuré pendant cette phase.

L'appareil est prêt pour un nouveau prélèvement.

Ce dispositif permet donc le nettoyage intérieur et extérieur de l'aiguille 3 même de grande longueur, à l'aide d'un boîtier de rinçage simplifié. Son encombrement est réduit et il s'escamote aisément dans le haut de l'appareil pendant un prélèvement. Le volume de fluide de rinçage est très réduit de même que le volume aspiré par le conduit 24 ; en outre aucun liquide ne peut se répandre sur le fond de l'appareil bien que la chambre 18 ne soit pas fermée à sa partie inférieure.

Le nettoyage intérieur de l'aiguille à contre-courant du sens de prélèvement du sang, combiné avec un nettoyage extérieur, grâce à l'injection complémentaire de fluide par le conduit 21, et un nettoyage extérieur de la partie supérieure de l'aiguille par les joints 16 donne de bien meilleurs résultats qu'avec les dispositifs connus.

Selon une disposition avantageuse l'aiguille 3 est inclinée sur la verticale ce qui rend plus facile l'introduction du flacon de prélèvement et sa manipulation. De plus l'aiguille 3 étant fixée sur son support 5 par l'étrier flottant 7, n'est donc pas rigidement maintenue et peut prendre certaines légères inclinaisons par rapport à sa position originale, lors du coulissement du boîtier 14, ce qui évite tout risque de coincement. Le support de boîtier étant parfaitement guidé par la colonne 9 et la bague 13, un vérin 10 de petite dimension et de faible puissance est suffisant pour la manoeuvre du boîtier. De même ce dernier peut-il coulisser facilement sur l'aiguille 3 grâce à la pièce de guidage 17 qui glisse sans frottement sur la paroi extérieure de l'aiguille.

## Revendications

1. Dispositif de nettoyage d'une aiguille (3) de prélèvement d'un liquide, notamment du sang, montée sur le bâti d'un appareil de prélèvement, à l'aide d'un boîtier de rinçage (14), comportant une chambre (18) cylindrique de nettoyage ouverte vers l'extérieur qui coulisse sur l'aiguille (3) fixe de prélèvement entre une position haute dans laquelle il dégage la partie inférieure de l'aiguille (3) et la laisse tout à fait accessible pour un prélèvement de liquide et une position basse qu'il occupe lors du nettoyage où il coiffe toute la partie inférieure de l'aiguille (3), l'extrémité de celle-ci restant en retrait à l'intérieur de la chambre (18) cylindrique caractérisé en ce que à la partie inférieure de ladite chambre (18) est appliquée en permanence une dépression par un orifice (23) qui aspire un fluide de rinçage injecté par le haut à l'intérieur de l'aiguille (3), lequel fluide assurant un rinçage intérieur, à contre-courant du sens de prélèvement du liquide, et en ce que une injection de fluide est prévue au sommet de la chambre (18) par un orifice additionnel (20) en vue d'assurer un nettoyage complémentaire extérieur de l'aiguille et sur toute sa hauteur, pendant la descente et pendant la remontée du boîtier de rinçage (14).

2. Dispositif de nettoyage selon la revendication 1, caractérisé en ce que le boîtier de rinçage (14) est fixé à un coulisseau (12) qui se déplace sur une colonne de guidage (9), fixe et parallèle à l'aiguille (3).

3. Dispositif de nettoyage selon les revendications 1 et 2, caractérisé en ce que la tige mobile (11) d'un vérin fixe (10) est solidaire du coulisseau (12).

4. Dispositif de nettoyage selon les revendications 2 et 3, caractérisé en ce qu'une même potence (8) fixée au bâti (6) de l'appareil de prélèvement, sert de support à la colonne (9) et au vérin (10)

5. Dispositif de nettoyage selon la revendication 1, caractérisé en ce que l'aiguille de prélèvement (3) est fixée, par l'intermédiaire d'un étrier flottant (7) à une équerre (5) solidaire du bâti (6) de l'appareil de prélèvement.

## Claims

1. Device for cleaning a needle (3) which is used for the taking off of liquid, especially blood, and which is mounted on the frame of a take-off apparatus, using a rinsing casing (14), comprising a cylindrical cleaning chamber (18) open towards the exterior, which slides on the fixed take-off needle (3) between an upper position wherein it frees the lower portion of the needle (3) and leaves it fully accessible for a take-off of liquid and a lower position which it occupies at the time of cleaning wherein it covers all the lower portion of the needle (3), the end of the latter remaining set back within the interior of the oylindrical chamber (18), characterised in that at the lower portion of the said chamber (18) a negative pressure is permanently applied through an orifice (23) which aspirates a rinsing fluid injected at the top into the needle (3), which fluid effects an internal rinsing in a direction opposite to the direction in which liquid is taken off, and in that fluid is injected at the top of the chamber (18) through an additional orifice (20) to effect an external complementary cleaning of the needle and over its entire height, during the descent and return ascent of the rinsing casing (14).

2. Cleaning device according to claim 1, characterised in that the rinsing casing (14) is secured to a slide (12) which is displaced on a guide column (9) which is fixed and parallel to the needle (3).

3. Cleaning device according to claims 1 and 2, characterised in that the movable rod (II) of a fixed jack (10) is integral with the slide (12).

4. Cleaning device according to claims 2 and 3, characterised in that one and the same bracket (8) secured to the frame (6) of the take-off apparatus serves as a support for the column (9) and for the jack (10).

5. Cleaning device according to claim 1, characterised in that the take-off needle (3) is secured, by means of a floating strap (7), to an angle element (5) integral with the frame (6) of the take-off apparatus.

**Ansprüche**

1. Reinigungsvorrichtung für eine Flüssigkeitsentnahmenadel (3), die, angebracht auf dem Gestell eines Gerätes für die Entnahme insbesondere von Blut, ein Gehäuse (14) zum Spülen mit einer zylindrischen Reinigungskammer (18) umfaßt, die nach außen offen ist, wobei das Gehäuse auf der festgehaltenen Entnahmenadel (3) gleiten kann zwischen einer oberen Stellung, in der der untere Teil der Nadel (3) vollständig für die Flüssigkeitsentnahme freigegeben ist, und einer unteren Stellung, die es während der Reinigung einnehmen kann, in der es den ganzen unteren Teil der Nadel (3) bedeckt, deren Spitze ins Innere der zylindrischen Kammer (18) zurückgezogen ruht, dadurch gekennzeichnet, daß im unteren Teil der genannten Reinigungskammer (18) eine Öffnung (23) angebracht ist, an der ständig ein Unterdruck herrscht und wo eine Spülflüssigkeit angesaugt werden kann, die von oben in die Nadel (3) eingebracht wird und die im Gegenlauf zur Entnahmeflüssigkeit für die Spülung des Nadel-Inneren sorgt, und dadurch, daß im obersten Teil der Reinigungskammer (18) eine weitere Öffnung (20) für die Zufuhr von Flüssigkeit vorgesehen ist, um für eine ergänzende Reinigung des Äußeren der Nadel in ihrer ganzen Höhe beim Auf- und Niedergang des Spülgehäuses (14) zu sorgen.

2. Reinigungsvorrichtung nach Anspruch 1, dadurch gekennzeichnet, daß das Spülgehäuse (14) an einem Gleitstück (12) befestigt ist, das sich an einer Führungssäule (9) bewegen kann, die fest und parallel zur Nadel (3) angebracht ist.

3. Reinigungsvorrichtung nach den Ansprüchen 1 und 2, dadurch gekennzeichnet, daß die bewegbare Kolbenstange (11) eines festeingebauten Druckzylinders (10) mit dem Gleitstück (12) fest verbunden ist.

4. Reinigungsvorrichtung nach den Ansprüchen 2 und 3, dadurch gekennzeichnet, daß der gleiche am Gestell (6) des Entnahmegerätes befestigte Stützträger (8) als Stütze für die Säule (9) und den Druckzylinder (10) dient.

5. Reinigungsvorrichtung nach Anspruch 1, dadurch gekennzeichnet, daß die Entnahmenadel (3) mittels eines schwimmenden Bügels (7) an einem Winkelstück (5) angebracht ist, das seinerseits an dem Gestell (6) des Entnamegerätes befestigt ist.

FIG.1

FIG. 2